# EUROPÄISCHE PATENTSCHRIFT

(11) **EP 0 609 738 B1**
(45) Veröffentlichungstag und Bekanntmachung des Hinweises auf die Patenterteilung: **27.05.1998**
(21) Anmeldenummer: 94100944.1
(22) Anmeldetag: 24.01.1994
(51) Int. Cl.: A61F 2/30, A61F 2/28, A61L 27/00

(54) **Abdeckeinrichtung für ein Knochenloch**
Bone hole covering device
Dispositif de recouvrement pour cavité osseuse

(30) Priorität: 01.02.1993 DE 4302709
(43) Veröffentlichungstag der Anmeldung: 10.08.1994
(73) Patentinhaber: Kirsch, Axel, Dr., 70794 Filderstadt (DE)
(72) Erfinder: Kirsch, Axel, Dr., 70794 Filderstadt (DE)
(74) Vertreter: Goddar, Heinz J., Dr.

(56) Entgegenhaltungen:
- EP-A- 0 475 077
- EP-A- 0 574 091
- WO-A-88/03417
- WO-A-88/05312
- DE-A- 3 042 003
- FR-A- 2 056 934
- GB-A- 1 430 071
- GB-A- 2 010 095

## Beschreibung

Die Erfindung betrifft eine gattungsgemäße Abdeckeinrichtung nach dem Oberbegriff von Anspruch 1.

In der Knochenchirurgie, beispielsweise bei der Rekonstruktion von Knochen in der plastischen Chirugie oder bei kieferchirugischen Operationen, ist es üblich, Knochendefektstellen in Form von Ausnehmungen oder Höhlungen im körpereigenen Knochengewebe mit Knochenaufbaumaterial zu füllen, welches in der Regel aus einer Mischung aus Knochenersatzmaterial, wie Hydroxylapatitgranulat, und körpereigenen Knochenpartikeln besteht. Um zu gewährleisten, daß das Knochenaufbaumaterial im wesentlichen ausschließlich von der Knochenseite her knöchern durchwachsen wird, nicht aber in unerwünschter Weise von Schleimhautgewebe, wird die Ausnehmung mit einer Abdeckmembran der eingangs genannten Art verschlossen, die verhindern soll, daß ein Durchwachsen des Knochenaufbaumaterials mit Nicht-Knochengewebe erfolgt. Nur dann nämlich, wenn ein vollständig knöchernes Durchwachsen des Knochenaufbaumaterials gewährleistet ist, läßt sich die Knochendefektstelle im wesentlichen vollständig beseitigen und das Knochenaufbaumaterial nach knöchernem Durchwachsen in den körpereigenen Knochen reintegrieren.

Bislang finden als Abdeckmembranen beispielsweise Polytetrafluorethylenfolien Verwendung, die jedoch den Nachteil haben, daß sie nach Ausheilen der Knochendefektstelle im Körper verbleiben und hierdurch zu Komplikationen Anlaß geben können.

Infolge des für Abdeckmembranen verwendeten Materials, wie z. B. Kunststoffolie, ist die Membran außerdem nicht steif, sondern kann sich infolge einer z. B. radialen Beanspruchung durch das umgebende weiche Körpergewebe, z. B. infolge von Muskelbewegungen, in ihrer Lage verändern. Derartige Bewegungen der Abdeckmembran haben den Nachteil, daß die Überführung des in der Knochendefektstelle befindlichen Blutkoagel in knochenspezifisch geprägtes Gewebe beeinträchtigt wird, weil nämlich die membrannahe Schicht des Knochenaufbaumaterials bzw. des Blutkoagels durch die Bewegung der Abdeckmembran in ihrer Bestimmung dedifferenziert wird.

WO-A-8803417 betrifft einen biologischen Verbundwerkstoff, der in der Knochenchirurgie zur Anwendung gelangen soll. Besagter Verbundwerkstoff umfaßt mindestens eine biokeramische Komponente und mindestens eine Materialkomponente, die aus mindestens einem Polymer hergestellt wurde und mit der biokeramischen Komponente mindestens eine gemeinsame Grenzfläche aufweist. Die Materialkomponente umfaßt mindestens Verstärkungselemente, die aus im wesentlichen resorbierbarem Material wie Polymer, Copolymer, Polymermischung und/oder keramischen Material hergestellt wurde und kann Bindematerial einschließen, das im wesentlichen aus resorbierbarem Polymer, Copolymer oder Polymermischung besteht. Die Materialkomponente weist zumindest im implantierten Zustand eine offene Porenstruktur auf.

Der Erfindung liegt die Aufgabe zugrunde, eine Abdeckeinrichtung der gattungsgemäßen Art zu schaffen, mittels welcher die ungestörte Überführung des Knochenaufbaumaterials bzw. des Blutkoagels in der Knochendefektstelle in Granulationsgewebe knochenspezifisch gepräger Art gewährleistet werden kann. Vorzugsweise soll durch entsprechende Auswahl des Membranmaterial für die Abdeckmembran auch vermieden werden, daß nach dem Ausheilen der Knochendefektstelle im Körper verbleibendes Membranmaterial zu Komplikationen Anlaß gibt.

Erfindungsgemäß wird diese Aufgabe durch die Merkmale des Kennzeichens von Anspruch 1 gelöst.

Bevorzugte Ausführungsformen der Erfindung ergeben sich aus den Unteransprüchen.

Weitere Merkmale und Vorteile der Erfindung ergeben sich aus den Ansprüchen und aus der nachstehenden Beschreibung, in der ein Ausführungsbeispiel anhand der schematischen Zeichnung im einzelnen erläutert ist. Dabei zeigt:
- Fig. 1: eine mit einer Abdeckeinrichtung nach einem Ausführungsbeispiel gemäß der Erfindung abgedeckte Knochendefektstelle im Schnitt senkrecht durch die Ebene der Abdeckeinrichtung; und
- Fig. 2: die Versteifungslage der Abdeckeinrichtung gemäß Figur 1 im Schnitt senkrecht zur Ebene der Abdeckmembran, in vergrößerter Darstellung.

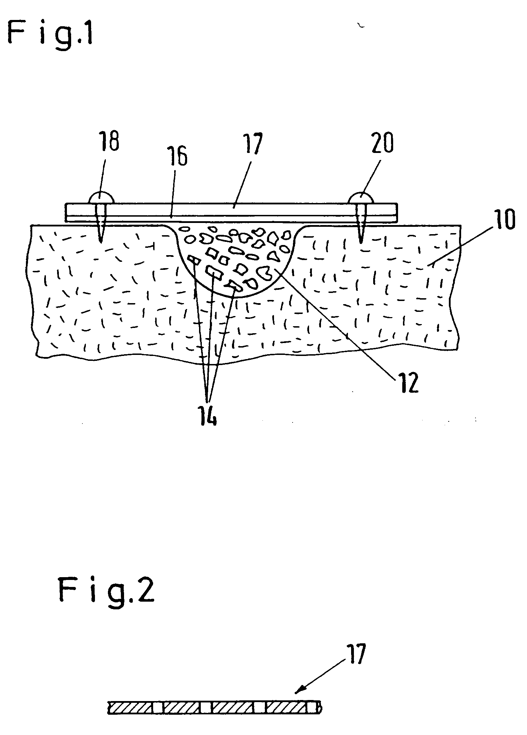

Wie Figur 1 erkennen läßt, ist dort eine in einem körpereigenen Knochen 10 durch eine Ausnehmung 12 gebildete Knochendefektstelle mit Knochenaufbaumaterial 14 aus Hydroxylapatitgranulat im wesentlichen vollständig gefüllt, wobei diesem Hydroxylapatitgranulat in bekannter Weise aus körpereigenem Knochengewebe bestehende Knochenpartikel beigemengt sind. Die mit dem Knochenaufbaumaterial 14 gefüllte Ausnehmung 12 ist von einer Abdeckmembran 16 und einer an diese auf der dem Knochen 10 abgewandten Seite angeordnete Versteifungslage 17 abgedeckt, wobei die Abdeckmembran 16 und die Versteifungslage 17 allseits der Ausnehmung 12 mittels Befestigungsnägeln 18, 20 in dichter Anlage am körpereigenen Knochen 10 befestigt sind. Die Abdeckmembran 16 hat den Zweck, das knöcherne Durchwachsen des Knochenaufbaumaterials 14 vom körpereigenen Knochen 10 her in der Weise zu gewahrleisten, daß das Durchwachsen des Knochenaufbaumaterials mit anderem als Knochengewebe, insbesondere Schleimhautgewebe verhindert wird.

Die Versteifungslage 17 besteht, wie Figur 2 erkennen laßt, aus einem perforierten Titanblech, dessen Dicke bei dem gezeigten Ausführungsbeispiel 0,5 mm beträgt. Das perforierte Metallblech der Versteifungslage 17 versteift die Abdeckmembran 16 derart, daß bei Bewegungen des den körpereigenen Knochens 10 umgebenden Bindegewebes etc. keine Relativbewegung der Abdeckmembran 16 bezüglich des Knochenaufbaumaterials 14 bzw. des die Ausnehmung 12 ausfüllenden Blutkoagels stattfindet, so daß letzteres ungestört und ohne die Gefahr einer unerwünschten Dedifferenzierung in knochenspezifisch geprägtes Gewebe überführt werden kann.

## Patentansprüche

1. Abdeckeinrichtung zum vortibergehenden Abdecken einer mit Knochenaufbaumaterial, wie Hydroxylapatitgranulat, gefüllten Ausnehmung in körpereigenem Gewebe, mit einer Abdeckmembran (16), die aus resorbierbarem Membranmaterial besteht und der eine Versteifungslage (17) zugeordnet ist, dadurch gekennzeichnet, daß das besagte Membranmaterial von der dem körpereigenen Knochen (10) abgewandten Seite schneller resorbierbar ist als von der dem körpereigenen Knochen (10) zugewandten Seite.

2. Abdeckeinrichtung nach Anspruch 1, dadurch gekennzeichnet, daß die Versteifungslage (17) in die Abdeckmembran (16) integriert ist.

3. Abdeckeinrichtung nach Anspruch 1, dadurch gekennzeichnet, daß die Versteifungslage (17) als von der Abdeckmembran (16) getrenntes Versteifungselement ausgebildet ist.

4. Abdeckeinrichtung nach einem der vorangehenden Ansprüche, dadurch gekennzeichnet, daß die Versteifungslage (17) an der dem körpereigenen Knochengewebe abgewandten Seite der Abdeckmembran (16) angeordnet ist.

5. Abdeckeinrichtung nach einem der vorangehenden Ansprüche, dadurch gekennzeichnet, daß die Versteifungslage (17) aus einem perforierten Metallblech besteht.

6. Abdeckeinrichtung nach einem der Ansprüche 1 bis 4, dadurch gekennzeichnet, daß die Versteifungslage (17) aus einem Metallgitter oder -netz besteht.

7. Abdeckeinrichtung nach Anspruch 5 oder 6, dadurch gekennzeichnet, daß die Versteifungslage (17) aus Titan hergestellt ist.

8. Abdeckeinrichtung nach einem der Ansprüche 1 bis 4, dadurch gekennzeichnet, daß die Versteifungslage (17) aus Kunststoff besteht.

9. Abdeckeinrichtung nach Anspruch 8, dadurch gekennzeichnet, daß die Versteifungslage (17) aus einem Kunststoffgitter besteht.

10. Abdeckeinrichtung nach Anspruch 8 oder 9, dadurch gekennzeichnet, daß das Kunststoffmaterial, aus dem die Versteifungslage (17) von dem die Abdeckmembran (16) bildenden Membranmaterial verschieden ist.

11. Abdeckeinrichtung nach einem der vorangehenden Ansprüche, dadurch gekennzeichnet, daß die Versteifungslage (17) eine Dicke von 0,3 bis 1,0 mm hat.

12. Abdeckeinrichtung nach Anspruch 11, dadurch gekennzeichnet, daß die Versteifungslage (17) eine Dicke von ca. 0,5 mm hat.

13. Abdeckeinrichtung nach einem der vorangehenden Ansprüche, dadurch gekennzeichnet, daß die Dichte des Membranmaterials (16) von der dem körpereigenen Knochen (10) abgewandten Seite zu der dem körpereigenen Knochen (10) zugewandten Seite zunimmt.

14. Abdeckeinrichtung nach einem der vorangehenden Ansprüche, dadurch gekennzeichnet, daß das Membranmaterial (16) aus mehreren Schichten unterschiedlicher Dichte und/oder Zusammensetzung besteht.

15. Abdeckeinrichtung nach Anspruch 14, dadurch gekennzeichnet, daß das Membranmaterial (16) mehrere gewebte oder gewirkte Schichten unterschiedlicher Textur aufweist.

16. Abdeckeinrichtung nach einem der vorangehenden Ansprüche, dadurch gekennzeichnet, daß das Membranmaterial (16) zumindest teilweise aus lyophilisierter Hirnhaut besteht.

17. Abdeckeinrichtung nach einem der Ansprüche 1 bis 15, dadurch gekennzeichnet, daß das Membranmaterial (16) zumindest teilweise aus Polylactid/Vicryl besteht.

18. Abdeckeinrichtung nach einem der Ansprüche 1 bis 15, dadurch gekennzeichnet, daß das Membranmaterial (16) zumindest teilweise aus Kollagen besteht.

19. Abdeckeinrichtung nach einem der Ansprüche 1 bis 15, dadurch gekennzeichnet, daß das Membranmaterial (16) zumindest teilweise aus Polylactid besteht.

20. Abdeckeinrichtung nach einem der Ansprüche 1 bis 15, dadurch gekennzeichnet, daß das Membranmaterial (16) zumindest teilweise aus Oxymethylcellulose besteht.

21. Abdeckeinrichtung nach einem der Ansprüche 1 bis 15, gekennzeichnet durch eine derartige Einstellung des von der dem körpereigenen Knochen (10) abgewandten Seite zu dem körpereigenen Knochen (10) zugewandten Seite abnehmenden Resorbierbarkeitsgradienten des Membranmaterials (16), daß die am Knochen (10) anliegende Schicht des Membranmaterials (16) erst dann von körpereigenem Gewebe resorbiert wird, wenn die durch die Abdeckmembran verschlossene Ausnehmung (12) des körpereigenen Knochens (10) im wesentlichen vollständig knöchern durchwachsen und in den Knochen (10) reintegriert ist.

## Claims

1. A covering device for temporarily covering a recess in endogenous tissue filled with a bone-forming material such as hydroxyl apatite granulate, comprising a covering membrane (16) consisting of resorbable membrane material and associated with a stiffening layer (17), characterised in that the said membrane material can be resorbed on the side remote from the endogenous bone (10) more quickly than on the side facing the endogenous bone (10).

2. A covering device according to claim 1, characterised in that the stiffening layer (17) is incorporated in the covering membrane (16).

3. A covering device according to claim 1, characterised in that the stiffening layer (17) is a stiffening element separate from the covering membrane (16).

4. A covering device according to any of the preceding claims, characterised in that the stiffening layer (17) is disposed on the side of the covering membrane remote from the endogenous bone tissue.

5. A covering device according to any of the preceding claims, characterised in that the stiffening layer (17) is a perforated metal plate.

6. A covering device according to any of claims 1 to 4, characterised in that the stiffening layer (17) comprises a metal grid or netting.

7. A covering device according to claim 5 or 6, characterised in that the stiffening layer (17) is made of titanium.

8. A covering device according to any of claims 1 to 4, characterised in that the stiffening layer (17) is of plastic.

9. A covering device according to claim 8, characterised in that the stiffening layer (17) comprises a plastic grid.

10. A covering device according to claim 8 or 9, characterised in that the plastic material forming the stiffening layer (17) is different from the material forming the covering membrane (16).

11. A covering device according to any of the preceding claims, characterised in that the stiffening layer (15) has a thickness of 0.3 to 1.0 mm.

12. A covering device according to claim 11, characterised in that the stiffening layer (17) has a thickness of about 0.5 mm.

13. A covering device according to any of the preceding claims, characterised in that the density of the membrane material (16) increases from the side remote from the endogenous bone (10) to the side facing the endogenous bone (10).

14. A covering device according to any of the preceding claims, characterised in that the membrane material (16) consists of a number of layers of different density and/or composition.

15. A covering device according to claim 14, characterised in that the membrane material (16) comprises a number of woven or knitted layers of different texture.

16. A covering device according to any of the preceding claims, characterised in that the membrane material (16) consists at least partly of freeze-dried meninges.

17. A covering device according to any of claims 1 to 15, characterised in that the membrane material (16) consists at least partly of polylactide/vicryl.

18. A covering device according to any of claims 1 to 15, characterised in that the membrane material (16) consists at least partly of collagen.

19. A covering device according to any of claims 1 to 15, characterised in that the membrane material (16) consists at least partly of polyactide.

20. A covering device according to any of claims 1 to 15, characterised in that the membrane material (16) consists at least partly of oxymethyl cellulose.

21. A covering device according to any of claims 1 to 15, characterised in that the resorbability gradient of the membrane material (16) from the side remote from the endogenous bone (10) to the side facing the endogenous bone (10) is such that the layer of membrane material (16) adjoining the bone (10) is not resorbed by the endogenous tissue until the recess (12) in the endogenous bone (10) closed by the covering membrane has been substantially completely intergrown and turned into bone and re-incorporated in the bone (10).

## Revendications

1. Dispositif de recouvrement pour le recouvrement provisoire d'une cavité dans du tissu appartenant au corps remplie d'une matière de constitution osseuse, telle que des granulés d'apatite d'hydroxyle, avec une membrane de recouvrement (16), qui se compose d'une matière de membrane apte à se résorber et à laquelle est associée une base raidissante (17), caractérisé en ce que ladite matière de membrane peut se résorber plus rapidement du côté opposé à l'os (10) appartenant au corps, que du côté orienté vers l'os (10) appartenant au corps.

2. Dispositif de recouvrement selon la revendication 1, caractérisé en ce que la base raidissante (17) est intégrée à la membrane de recouvrement (16).

3. Dispositif de recouvrement selon la revendication 1, caractérisé en ce que la base raidissante (17) est conçue comme élément de raidissement séparé de la membrane de recouvrement (16).

4. Dispositif de recouvrement selon l'une quelconque des revendications précédentes, caractérisé en ce que la base raidissante (17) est disposée sur le côté de la membrane de recouvrement (16) opposé au tissu osseux appartenant au corps.

5. Dispositif de recouvrement selon l'une quelconque des revendications précédentes, caractérisé en ce que la base raidissante (17) se compose d'une plaque métallique perforée.

6. Dispositif de recouvrement selon l'une des revendications 1 à 4, caractérisé en ce que la base raidissante (17) se compose d'une grille métallique ou d'un maillage métallique.

7. Dispositif de recouvrement selon la revendication 5 ou 6, caractérisé en ce que la base raidissante (17) est fabriquée à partir de titane.

8. Dispositif de recouvrement selon l'une des revendications 1 à 4, caractérisé en ce que la base raidissante (17) se compose de plastique.

9. Dispositif de recouvrement selon la revendication 8, caractérisé en ce que la base raidissante (17) se compose d'une grille de plastique.

10. Dispositif de recouvrement selon la revendication 8 ou 9, caractérisé en ce que la matière plastique de la base raidissante (17) est différente de la matière de membrane constituant la membrane de recouvrement (16).

11. Dispositif de recouvrement selon l'une quelconque des revendications précédentes, caractérisé en ce que la base raidissante (17) présente une épaisseur comprise entre 0,3 et 1,0 mm.

12. Dispositif de recouvrement selon la revendication 11, caractérisé en ce que la base raidissante (17) présente une épaisseur d'environ 0,5 mm.

13. Dispositif de recouvrement selon l'une quelconque des revendications précédentes, caractérisé en ce que la densité de la matière de la membrane (16) augmente depuis le côté opposé à l'os (10) appartenant au corps vers le côté orienté vers l'os (10) appartenant au corps.

14. Dispositif de recouvrement selon l'une quelconque des revendications précédentes, caractérisé en ce que la matière de la membrane (16) se compose de plusieurs couches de densités et/ou de compositions diverses.

15. Dispositif de recouvrement selon la revendication 14, caractérisé en ce que la matière de la membrane (16) présente plusieurs couches tissées ou tricotées de textures diverses.

16. Dispositif de recouvrement selon l'une quelconque des revendications précédentes, caractérisé en ce que la matière de la membrane (16) se compose au moins pour partie de méninge lyophilisée.

17. Dispositif de recouvrement selon l'une des revendications 1 à 15, caractérisé en ce que la matière de la membrane (16) se compose au moins pour partie de polylactide/vicryle.

18. Dispositif de recouvrement selon l'une des revendications 1 à 15, caractérisé en ce que la matière de la membrane (16) se compose au moins pour partie de collagène.

19. Dispositif de recouvrement selon l'une des revendications 1 à 15, caractérisé en ce que la matière de la membrane (16) se compose au moins pour partie de polylactide.

20. Dispositif de recouvrement selon l'une des revendications 1 à 15, caractérisé en ce que la matière de la membrane (16) se compose au moins pour partie d'oxyméthylcellulose.

21. Dispositif de recouvrement selon l'une des revendications 1 à 15, caractérisé par un ajustement tel du gradient de résorbabilité de la matière de la membrane (16) décroissant depuis le côté opposé à l'os (10) appartenant au corps vers le côté orienté vers l'os (10) appartenant au corps, que la couche de la matière de la membrane (16) reposant sur l'os (10) n'est résorbée par le tissu appartenant au corps que lorsque la cavité (12) de l'os (10) appartenant au corps fermée par la membrane de recouvrement a réalisé une interpénétration pour l'essentiel complète sur le plan osseux et s'est réintégrée dans l'os (10).
